# EUROPÄISCHE PATENTSCHRIFT

(11) **EP 0 648 104 B1**
(45) Veröffentlichungstag und Bekanntmachung des Hinweises auf die Patenterteilung: **07.01.1998**
(21) Anmeldenummer: 93914725.2
(22) Anmeldetag: 24.06.1993
(51) Int. Cl.: A61K 7/06

(54) **HAARBEHANDLUNGSMITTEL**
HAIR-TREATMENT AGENTS
AGENTS DE TRAITEMENT CAPILLAIRE

(30) Priorität: 03.07.1992 DE 4221914; 13.10.1992 DE 4234413
(43) Veröffentlichungstag der Anmeldung: 19.04.1995
(73) Patentinhaber: Henkel Kommanditgesellschaft auf Aktien, 40191 Düsseldorf (DE)
(72) Erfinder: SEIDEL, Kurt, D-4000 Düsseldorf 13 (DE); MÜLLER, Reinhard, D-5140 Erkelenz 7 (DE); HOLLENBERG, Detlef, D-4006 Erkrath (DE); PRIEBE, Christian, D-5603 Wülfrath (DE)
(86) Internationale Anmeldenummer: EP9301626
(87) Internationale Veröffentlichungsnummer: WO9401077

(56) Entgegenhaltungen:
- WO-A-91/03229
- WO-A-92/17153
- GB-A- 2 063 671
- GB-A- 2 098 624
- GB-A- 2 113 245
- GB-A- 2 134 784
- GB-A- 2 136 689

## Beschreibung

Die Erfindung betrifft Zubereitungen zur Haarbehandlung mit einer speziellen Kombination von Polymeren.

Die Reinigung und Pflege der Haare ist ein wichtiger Bestandteil der menschlichen Körperpflege. Sowohl die Reinigung der Haare mit Shampoos als auch die dekorative Gestaltung der Frisur beispielsweise durch Färben oder Dauerwellen sind Eingriffe, die die natürliche Struktur und die Eigenschaften der Haare beeinflussen. So können anschließend an eine solche Behandlung beispielsweise die Naß- und Trockenkämmbarkeit des Haares, der Halt und die Fülle des Haares unbefriedigend sein oder die Haare einen erhöhten Spliß aufweisen.

Es ist daher seit langem üblich, die Haare einer speziellen Nachbehandlung zu unterziehen. Dabei werden, üblicherweise in Form einer Spülung, die Haare mit speziellen Wirkstoffen, beispielsweise quaternären Ammoniumsalzen oder speziellen Polymeren, behandelt. Durch diese Behandlung werden je nach Formulierung Kämmbarkeit, Halt und Fülle der Haare verbessert und die Splißrate verringert.

Zusätze von kationischen Polymeren zu Haarbehandlungsmitteln führen in der Regel zu einer verbesserten Naß- und Trockenkämmbarkeit; Zusätze von amphoteren Polymeren zu einer stark verbesserten Naßkämmbarkeit, während die Trockenkämmbarkeit meist nur wenig beeinflußt wird.

Weitere positive Effekte lassen sich häufig durch Kombination von mehreren Wirkstoffen aus unterschiedlichen Klassen erzielen. So ist beispielsweise aus der deutschen Offenlegungsschrift DE-A1-30 44 738 bekannt, Haarbehandlungsmittel mit einer Mischung aus amphoteren und kationischen Polymeren zu versehen.

Weiterhin sind aus der GB 2,113,245 Zusammensetzungen zur Behandlung von Keratinfasern bekannt, die amphotere Polymere mit Betaingruppen in Kombination mit kationischen Verbindungen enthalten. Als kationische Verbindungen werden u. a. Polymere mit quartären Ammoniumgruppen, wie z. B. das Handelsprodukt Luviquat FC 905, genannt. WO 91/03229 offenbart zwitterionische Polymerisate aus bestimmten kationischen und bestimmten anionischen Polymeren, die in Zubereitungen zur Haarbehandlung gemeinsam mit weiteren Polymeren enthalten sein können.

Während die Verbesserung der Naßkämmbarkeit, d.h. eine Erniedrigung der Naßkämmarbeit, in allen Fällen gewünscht wird, sind die Verhältnisse bei der Trockenkämmbarkeit komplizierter. Niedrige Kämmarbeits-Werte charakterisieren zwar eine Verbesserung der Kämmbarkeit; wird die Kämmarbeit aber zu sehr erniedrigt, so verliert das Haar Fülle und Halt, so daß sich im Extremfall bestimmte Frisuren nicht mehr aufbauen lassen. Daher kann, vor allem bei komplexeren Frisuren, eine in bestimmten Grenzen erhöhte Trockenkämmarbeit durchaus gewünscht sein, um den Halt der Frisur zu verbessern.

Es wurde nun überraschenderweise gefunden, daß Mittel mit einer Kombination aus bestimmten amphoteren oder zwitterionischen Polymeren und bestimmten kationischen Polymeren die Naßkämmbarkeit in gewünschter Weise verbessern, während die Trockenkämmarbeit in einen Bereich gesteigert wird, der für den Halt der Frisur optimal ist. Somit wird sowohl eine deutliche Verbesserung der Frisierbarkeit im feuchten Zustand, als auch eine Steigerung des Frisurenhalts im trockenen Zustand erreicht. Darüber hinaus zeigen die behandelten Haare ein gutes Lockenrückhaltevermögen und eine gute Sprungkraft.

Gegenstand der Erfindung sind daher wäßrige Zubereitungen zur Behandlung von Haaren, enthaltend neben üblichen kosmetischen Bestandteilen eine Kombination von kationischen und amphoteren oder zwitterionischen Polymeren, dadurch gekennezeichnet, daß die kationischen Polymeren Imidazoliniumgruppen enthalten sowie mindestens ein nichtionisches Monomer, wobei das Verhältnis zwischen kationischen und nichtionischen Monomeren im Bereich 1,5:1 bis 1:1,5 liegt, und sich die zwitterionischen Polymeren im wesentlichen zusammensetzen aus
α) Monomeren mit quartären Ammoniumgruppen der allgemeinen Formel (I),

   R¹-CH=CR²-CO-Z-(CₙH₂ₙ)-N⁽⁺⁾R³R⁴R⁵ A⁽⁻⁾ (I)

   in der R¹ und R² unabhängig voneinander stehen für Wasserstoff oder eine Methylgruppe und R³, R⁴ und R⁵ unabhängig voneinander für Alkylgruppen mit 1 bis 4 Kohlenstoffatomen, Z eine NH-Gruppe oder ein Sauerstoffatom, n eine ganze Zahl von 2 bis 5 und A⁽⁻⁾ das Anion einer organischen oder anorganischen Säure ist
   und
β) monomeren Carbonsäuren der allgemeinen Formel (II),

   R⁶-CH=CR⁷-COOH (II)

   in denen R⁶ und R⁷ unabhängig voneinander Wasserstoff oder Methylgruppen sind, oder Alkali-, Erdalkali,- Aluminium- oder Ammoniumsalzen dieser Säuren.

Die erfindungsgemäß verwendbaren kationischen Polymeren sind aus Verbindungen aufgebaut, die neben mindestens einer kationischen Gruppe mindestens eine polymerisierbare Gruppe enthalten und frei sind von anionischen Gruppen.

Die polymerisierbare Gruppe ist bevorzugt eine Vinylgruppe. Es sind jedoch auch kationische Polymerisate verwendbar, bei denen die Polymerhauptkette beispielsweise aus Glykosiden aufgebaut ist.

Übliche kationische Gruppen sind Gruppen mit quartären Stickstoff- oder Phosphoratomen. Gruppen mit quartären Stickstoffatomen sind dabei bevorzugt. Die quartären Stickstoffatome können dabei sowohl 4 unterschiedliche oder z.T. gleiche Substituenten tragen, als auch Teil eines Ringsystems sein. Für die vorliegende Erfindung ist wesentlich, daß zumindest ein Teil der kationischen Gruppen quartäre Stickstoffatome enthält, die Teil eines Imidazoliniumringes sind. Polymere, bei denen mindestens 50 % der kationischen Gruppen Imidazolinium-Ringe enthalten, sind erfindungsgemäß bevorzugt. Besonders bevorzugt sind solche Polymere, bei denen die Imidazoliniumsysteme die einzigen kationischen Gruppen sind.

Die Imidazoliniumsysteme sind bevorzugt quaternierte Imidazole, die lediglich die polymerisierbare Gruppe als Substituenten enthalten. Es können jedoch auch solche Imidazole eingesetzt werden, die weitere Substituenten, insbesondere Alkylgruppen mit 1 bis 4 Kohlenstoffatomen, enthalten. Die Quaternierung kann mit allen bekannten Quaternierungsmitteln, beispielsweise Dimethylsulfat, Diethylsulfat und Methylchlorid, erfolgen.

Weiterhin enthalten die kationischen Polymeren neben den kationischen Monomeren noch mindestens ein nichtionisches Monomer, wobei das Verhältnis zwischen kationischen und nichtionischen Monomeren im Bereich 1,5:1 bis 1:1,5 liegt. Copolymere, bei denen das Verhältnis von kationischen und nichtionischen Monomeren etwa bei 1:1 lag, haben sich als erfindungsgemäß besonders geeignet erwiesen. Geeignete nichtionische Monomere sind beispielsweise Vinylpyrrolidon, Vinylacetat, Acrylamid, Methacrylamid, Methylacrylat, Ethylacrylat, Methylmethacrylat und Ethylmethacrylat. Vinvlpyrrolidon ist ein besonders bevorzugtes nichtionisches Monomer.

Vinylimidazoliniummethochlorid/Vinylpyrrolidon-Copolymerisate sind beispielsweise unter der Bezeichnung Luviquat^{R} (BASF) kommerziell erhältlich.

Unter "amphoteren Polymeren" sollen im Sinne der Erfindung Polymere verstanden werden, die im Molekül sowohl freie Aminogruppen als auch freie -COOH- oder -SO₃H-Gruppen enthalten und zur Ausbildung innerer Salze befähigt sind. Unter "zwitterionischen Polymeren" werden solche Polymeren verstanden, die im Molekül quartäre Ammoniumgruppen und -COO⁻- oder -SO₃⁻-Gruppen enthalten.

Ein Beispiel für ein erfindungsgemäß einsetzbares amphoteres Polymer ist das unter der Bezeichnung Amphomer^{R} erhältliche Acrylharz, das ein Copolymeres aus tert.-Butylaminoethylmethacrylat, N-(1,1,3,3-Tetramethylbutyl)acrylamid sowie zwei oder mehr Monomeren aus der Gruppe Acrylsäure, Methacrylsäure und deren einfachen Estern darstellt.

Die erfindungsgemäß verwendeten zwitterionischen Polymere setzen sich im wesentlichen zusammen aus
(α) Monomeren mit quartären Ammoniumgruppen der allgemeinen Formel (I),

   R¹-CH=CR²-CO-X-(CₙH₂ₙ)-N⁽⁺⁾R³R⁴R⁵ A⁽⁻⁾ (I)

   in der R¹ und R² unabhängig voneinander stehen für Wasserstoff oder eine Methylgruppe und R³, R⁴ und R⁵ unabhängig voneinander für Alkylgruppen mit 1 bis 4 Kohlenstoff-Atomen, X eine NH-Gruppe oder ein Sauerstoffatom, n eine ganze Zahl von 2 bis 5 und A⁽⁻⁾ das Anion einer organischen oder anorganischen Säure ist
   und
(β) monomeren Carbonsäuren der allgemeinen Formel (II),

   R⁶-CH=CR⁷-COOH (II)

   in denen R⁶ und R⁷ unabhängig voneinander Wasserstoff oder Methylgruppen sind.

Diese Verbindungen können sowohl direkt als auch in Salzform, die durch Neutralisation der Polymerisate, beispielsweise mit einem Alkalihydroxid, erhalten wird, erfindungsgemäß eingesetzt werden. Bezüglich der Einzelheiten der Herstellung dieser Polymerisate wird ausdrücklich auf den Inhalt der deutschen Offenlegungsschrift 39 29 973 Bezug genommen.

Ganz besonders bevorzugt sind solche Polymeren auf Basis von Monomeren des Typs (α), bei denen R³, R⁴ und R⁵ Methylgruppen sind, X eine NH-Gruppe und A⁽⁻⁾ ein Halogenid-, Methoxysulfat- oder Ethoxysulfat-Ion ist; Acrylamidopropyltrimethylammoniumchlorid ist ein besonders bevorzugtes Monomeres (α). Als Monomeres (β) für die genannten Polymeren wird bevorzugt Acrylsäure oder ein Alkalisalz der Acrylsäure, insbesondere das Natriumsalz, verwendet.

Weiterhin sind solche zwitterionischen Polymere bevorzugt, bei denen die Zahl der Monomeren vom Typ (α) größer als die Zahl der Monomeren vom Typ (β) ist. Momomerenverhältnisse (α):(β) größer als 1,5 sind besonders bevorzugt.

Sowohl kationische als auch amphotere und zwitterionische Polymere sind bevorzugt in Mengen von 0,01 - 10 Gew.-%, bezogen auf die gesamte Zubereitung, enthalten. Mengen von 0,01 bis 5 Gew.-% sind besonders bevorzugt.

Zubereitungen, die kationische Polymere und amphotere oder zwitterionische Polymere in Mengenverhältnissen zwischen 10:1 und 2:1 enthalten, haben sich als besonders wirksam erwiesen.

Es wurde ferner gefunden, daß besonders gute Ergebnisse im Sinne der erfindungsgemäßen Lehre erzielt werden, wenn die erfindungsgemäßen Zubereitungen zusätzlich ein Tensid enthalten. Es kann sich dabei um ein anionisches, kationisches, ampholytisches, zwitterionisches oder nichtionisches Tensid handeln.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil^{R}-Quat 3270 und 3272 (Hersteller: Th. Goldschmidt; diquaternäre Polydimethylsiloxane, Quaternium-80).

Alkylamidoamine, insbesondere Fettsäureamidoamine wie das unter der Bezeichnung Tego Amid^{R}S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats", wie die unter dem Warenzeichen Stepantex^{R} vertriebenen Dialkylammoniummethosulfate und Methyl-hydroxyalkyl-dialkoyloxyalkyl-ammoniummethosulfate.

Ein Beispiel für ein als kationisches Tensid einsetzbares quaternäres Zuckerderivat stellt das Handelsprodukt Glucquat^{R}100 dar, gemäß CTFA-Nomenklatur ein "Lauryl Methyl Gluceth-10 Hydroxypropyl Dimonium Chloride".

Besonders bevorzugte kationische Tenside sind Alkylamidoamine, quaternäre Esterverbindungen und quaternäre Zuckerderivate.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ-CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel RKO(CH₂-CH₂O)ₓ-OSO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykol-ether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül, sowie Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Unter ampholytischen Tensiden werden solche oberflächenaktiven Verbindungen verstanden, die außer einer C₈-C₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂-₁₈-Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Glycerin,
- C₈-C₂₂-Alkylmono- und -oligoglycoside und deren ethoxylierte Analoga sowie
- Anlagerungsprodukte von 5 bis 60 Mol Ethylenoxid an Rizinusöl und gehärtetes Rizinusöl.

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Die Tensid-Menge, die in den erfindungsgemäßen Zubereitungen enthalten sein kann, hängt von der Art des Tensids und dem Verwendungszweck der Zubereitung ab.

Haarnachbehandlungsmittel enthalten bevorzugt kationische und/oder nichtionische Tenside, insbesondere in Mengen von 0,1 bis 10 Gew.-%, bezogen auf die gesamte Zubereitung. Mengen zwischen 0,1 und 3 Gew.-% sind besonders bevorzugt.

Haarkuren enthalten ebenfalls bevorzugt kationische und/oder nichtionische Tenside, insbesondere in Mengen von 0,1 bis 10 Gew.-%, bezogen auf die gesamte Zubereitung. Mengen zwischen 0,1 und 5 Gew.-% sind besonders bevorzugt.

Shampoos enthalten bevorzugt anionische Tenside, gewünschtenfalls in Kombination mit ampholytischen und/oder nichtionischen Tensiden, insbesondere in Mengen von 1 bis 50 Gew.-%, bezogen auf die gesamte Zubereitung. Mengen zwischen 3 und 20 Gew.-% sind besonders bevorzugt.

Die erfindungsgemäßen Zubereitungen weisen bevorzugt pH-Werte von 2,5 bis 7, insbesondere von 3 bis 6, auf.

Shampoos, Haarnachbehandlungsmittel und Haarkuren sind bevorzugte erfindungsgemäße Zubereitungen. Gleichwohl kann es sich bei den Zubereitungen auch um Dauerwellpräparate, Färbemittel, Fönwellen und andere übliche Haarbehandlungsmittel handeln.

Die Zubereitungen können als wäßrige oder wäßrig-alkoholischen Lösungen, Cremes, Lotionen, Gele, Emulsionen sowie anderen, in der Kosmetik üblichen Konfektionierungsformen formuliert sein.

Entsprechend der Art des Haarbehandlungsmittels und der Konfektionierungsform können die Zubereitungen alle bekannten weiteren Bestandteile enthalten.

Solche üblichen Bestandteile sind:
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere/Acrylsäure/Ethylacrylat/N-tert.Butylacrylamid-Terpolymere,
- weitere kationische Polymere wie beispielsweise quaternisierte Celluloseether, Polysiloxane mit quaternären Gruppen, Acrylamid/Dimethyldiallylammoniumchlorid-Copolymere sowie mit Diethylsulfat quaternierte Dimethylaminomethacrylat/Vinylpyrrolidon-Copolymere,
- Verdickungsmittel wie Agar-Agar, Guar-Gum, Alginate, Xanthan-Gum und Cellulose-Derivate,
- Strukturanten wie Glucose und Maleinsäure,
- haarkonditionierende Verbindungen wie Phospholipide, beispielsweise Sojalecithin, Ei-Lecitin und Kephaline, sowie Silikonöle,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsvermittler, wie Ethanol, Isopropanol, Ethylenglykol, Propylenglykol, Glycerin und Diethylenglykol,
- Farbstoffe,
- Antischuppenwirkstoffe wie Piroctone Olamine und Zink Omadine,
- weitere Substanzen zur Einstellung des pH-Wertes,
- Wirkstoffe wie Panthenol, Allantoin, Pyrrolidoncarbonsäuren und deren Salze, Pflanzenextrakte und Vitamine,
- Lichtschutzmittel,
- Konsistenzgeber wie Zuckerester, Polyolester oder Polyolalkylether,
- Fette und Wachse, wie Walrat, Bienenwachs, Montanwachs, Paraffine und Fettalkohole,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex,
- Perlglanzmittel wie Ethylenglykolmono- und -distearat,
- Treibmittel wie Propan-Butan-Gemische, N₂O, Dimethylether, CO₂ und Luft sowie
- Antioxidantien.
- direktziehende Farbstoffe
- sogenannte Kuppler- und Entwicklerkomponenten als Oxidationsfarbstoffvorprodukte,
- Reduktionsmittel wie z.B. Thioglykolsäure und deren Derivate, Thiomilchsäure, Cysteamin, Thioäpfelsäure und µ-Mercaptoethansulfonsäure,
- Oxidationsmittel wie Wasserstoffperoxid, Kaliumbromat und Natriumbromat.

Ebenfalls Gegenstand der Erfindung ist ein Verfahren zur Behandlung von Haaren mit einer erfindungsgemäßen Zubereitung.

Bevorzugt ist ein solches Verfahren, bei dem Zubereitungen mit der erfindungsgemäßen Wirkstoffkombination auf das Haar aufgebracht werden und nach einer gewissen Einwirkzeit, die in der Regel zwischen einigen Sekunden und ca. 20 Minuten liegt, mit Wasser oder einem im wesentlichen Wasser enthaltenden Mittel wieder vom Haar abgespült werden.

Die folgenden Beispiele sollen die Erfindung näher erläutern.

### Beispiele

### I. Bestimmung von Naß- und Trockenkämmbarkeit

### Untersuchungsmethoden

Den Untersuchungen zur Kämmbarkeit wurde die Methode gemäß J. Soc. Cosm. Chem. 1973 [24] 782 zugrundegelegt.

Es wurde jeweils die Kämmarbeit an braunem Haar (Alkinco #6634, Strähnenlänge 12 cm, Strähnenmasse 1 g) untersucht. Es handelte sich um leicht vorgeschädigte (kaltgewellte bzw. blondierte) Haare, wie sie etwa beim durchschnittlichen Anwender zu erwarten sind. Nach der Nullmessung wurden die Strähnen mit 100 ml der zu prüfenden Rezeptur getränkt. Nach 5 Minuten Einwirkzeit wurden die Strähnen 1 Minute unter fließendem Wasser (1 l/min, 38 °C) ausgespült. Zur Bestimmung der Naßkämmarbeit wurden die Strähnen dann erneut vermessen. Zur Bestimmung der Trockenkämmarbeit wurden die Strähnen zunächst 12 Stunden bei 30 °C und einer relativen Luftfeuchtigkeit von 20% getrocknet und dann erneut vermessen.

### Ergebnisse

Die Zusammensetzung der untersuchten Mischungen sowie die Ergebnisse der Kämmarbeitsmessungen sind in Tabelle 1 zusammengestellt. Die Werte für die Kämmarbeiten sind jeweils auf den Wert der Nullmessung bezogen; ihre statistische Sicherheit betrug 99,99%.

**Tabelle 1**

| [Mengenangaben in Gewichtsteilen] | | | | | | |
|---|---|---|---|---|---|---|
| Komponente / Mischung | V1 | V2 | V3 | E1 | V4 | V5 |
| Stenol^{R}1618¹ | 3,0 | 3,0 | 3,0 | 3,0 | 3,0 | 3,0 |
| Cutina^{R}GMS² | 0,5 | 0,5 | 0,5 | 0,5 | 0,5 | 0,5 |
| Eumulgin^{R}B 1³ | 0,4 | 0,4 | 0,4 | 0,4 | 0,4 | 0,4 |
| Eumulgin^{R}B 2⁴ | 0,8 | 0,8 | 0,8 | 0,8 | 0,8 | 0,8 |
| Eutanol^{R}G⁵ | 1,0 | 1,0 | 1,0 | 1,0 | 1,0 | 1,0 |
| Acrylamidopropyltrimethylammoniumchlorid/Acrylsäure-Copolymeres, mit Natronlauge neutralisiert (Polymer P1, gemäß DE 39 29 973) | - | 0,2 | - | 0,2 | - | 0,2 |
| Luviquat^{R}FC 550⁶ | - | - | 4,0 | 4,0 | - | - |
| Gafquat^{R}755-N⁷ | - | - | - | - | 8,0 | 8,0 |
| Wasser | <-------------------ad 100 ------------------> | | | | | |
| Naßkämmarbeit [%] - kaltgewellte Haare - | 85 | 47 | 76 | 69 | 74 | 70 |
| Trockenkämmarbeit [%] - kaltgewellte Haare - | 152 | 85 | 210 | 266 | 152 | 165 |
| Naßkämmarbeit [%] - blondierte Haare- | 84 | | | 70 | | |
| Trockenkämmarbeit [%] - blondierte Haare - | 95 | | | 236 | | |

| | | | | | | |
|---|---|---|---|---|---|---|
| ¹ C16/C18-Fettalkohol (HENKEL) | | | | | | |
| ² Glycerinmonostearat (CTFA-Bezeichnung: Glyceryl Stearate) (HENKEL) | | | | | | |
| ³ Cetylstearylalkohol mit ca. 12 Mol EO (CTFA-Bezeichhung: Ceteareth-12) (HENKEL) | | | | | | |
| ⁴ Cetylstearylalkohol mit ca. 20 Mol EO (CTFA-Bezeichnung: Ceteareth-20) (HENKEL) | | | | | | |
| ⁵ Kondensationsprodukt aus gesättigten flüssigen Fettalkoholen, vorwiegend Decylalkohol, hergestellt nach der Guerbet-Reaktion (CTFA-Bezeichnung: Octyldodecanol) (HENKEL) | | | | | | |
| ⁶ Vinylimidazoliniummethochlorid/Vinylpyrrolidon-Copolymerisat (50:50) (mittleres Molekulargewicht: ca. 80000 Dalton; 40 % Aktivsubstanz; CTFA-Bezeichnung: Polyquaternium-16) (BASF) | | | | | | |
| ⁷ Vinylpyrrolidon/Dimethylaminoethylmethacrylat-Copolymer, mit Diethylsulfat quaterniert (19 % Aktivsubstanz in Wasser) (GAF) | | | | | | |

Die Ergebnisse zeigen, daß die Anwendung der erfindungsgemäßen Mischungen zu der gewünschten Verbesserung der Naßkämmarbeit (der anzustrebende Bereich liegt bei Werten um und kleiner 70) bei gleichzeitigem Anstieg der Trockenkämmarbeit in den für den Frisurenhalt optimalen Bereich von ca. 230-270 führt.

### II. Anwendungsbeispiele

Die Mengenangaben in den folgenden Beispielen sind Gew.-%.

| 1) Haarspülung | |
|---|---|
| Stenol^{R}1618 | 1,8 |
| Tegoamid^{R}S 18⁸ | 1,6 |
| 1,2-Propylenglykol | 1,0 |
| Zitronensäure | 0,6 |
| Luviquat^{R}FC 550 | 1,0 |
| Polymer P1, gemäß DE 39 29 973 | 0,2 |
| Parfümöl, Wasser | ad 100 |

| | |
|---|---|
| ⁸ N,N-Dimethyl-N'-stearoyl-1,3-diamino-propan (CTFA-Bezeichnung: Stear amidopropyl Dimethylamin (GOLDSCHMIDT) | |

| 2) Haarspülung | |
|---|---|
| Stenol^{R}1618 | 1,8 |
| Stepantex^{R} VS 90⁹ | 1,8 |
| 1,2-Propylenglykol | 0,7 |
| Zitronensäure | 0,2 |
| Luviquat^{R}HM 552^{6a} | 2,5 |
| Polymer P1, gemäß DE 39 29 973 | 0,4 |
| Parfümöl, Farbstoff, Wasser | ad 100 |

| | |
|---|---|
| ⁹ N-Methyl-N(2-hydroxyethyl)-N,N-di(talgacyloxyethyl)ammoniummethosulfat (90% Aktivsubstanz in Isopropanol) (STEPAN) | |
| ^{6a} Vinylimidazoliniummethochlorid/Vinylpyrrolidon-Copolymerisat (50:50) (Mittleres Molekulargewicht: ca. 800.000 Dalton; 20 % Aktivsubstanz; CTFA-Bezeichnung: Polyquaternium 16) (BASF) | |

| 3) Haarspülung | |
|---|---|
| Rewoquat^{R}W 7500¹⁶ | 1,1 |
| Lanette^{R}O¹⁷ | 3,0 |
| Eumulgin^{R}B 1 | 0,8 |
| Eumulgin^{R}B 2 | 1,6 |
| Cutina^{R}GMS | 0,5 |
| Eutanol^{R}G | 1,0 |
| Luviquat^{R}HM 552 | 1,2 |
| Polymer P1, gemäß DE 39 29 973 | 0,4 |
| Wasser | ad 100 |

| | |
|---|---|
| ¹⁶ 1-Methyl-2-nortalgalkyl-3-talgfettsäure-amidoethyl-imidazoliniummethosulfat (ca. 75 % Aktivsubstanz) (REWO) | |
| ¹⁷ Gemisch höherer, gesättigter Fettalkohole, vorwiegend Cetyl- und Stearylalkohol (CTFA-Bezeichnung: Cetearyl Alcohol) (HENKEL) | |

| 4) Shampoo | |
|---|---|
| Texapon^{R}N 25¹⁸ | 43,0 |
| Dehyton^{R}K¹⁹ | 10,0 |
| Plantaren^{R}-1200²⁰ | 4,0 |
| Euperlan^{R}PK 3000²¹ | 1,6 |
| Arquad^{R}316²² | 0,5 |
| Luviquat^{R}FC 550 | 1,2 |
| Polymer P1, gemäß DE 39 29 973 | 0,2 |
| Glucamate^{R}DOE 120²³ | 0,5 |
| Natriumchlorid | 0,2 |
| Wasser | ad 100 |

| | |
|---|---|
| ¹⁸ Natriumlaurylethersulfat (ca. 28 % Aktivsubstanz; CTFA-Bezeichnung: Sodium Laureth Sulfate) (HENKEL) | |
| ¹⁹ Fettsäureamid-Derivat mit Betainstruktur der Formel R-CONH(CH₂)₃N⁺(CH₃)₂CH₂COO⁻ (ca. 30% Aktivsubstanz; CTFA-Bezeichnung Cocoamidopropyl Betaine) (HENKEL) | |
| ²⁰ C12-C16-Alkylglucosid mit Oligomerisationsgrad 1,4 (ca. 50 % Aktivsubstanz; CTFA-Bezeichnung: Lauryl Polyglycosid) (HENKEL) | |
| ²¹ Flüssige Dispersion von perlglanzgebenden Substanzen und Amphotensid (ca. 62 % Aktivsubstanz; CTFA-Bezeichnung: Glycol Distearate (and) Glycerin (and) Laureth-4 (and) Cocoamidopropyl Betaine) (HENKEL) | |
| ²² Tri-C16-alkylmethylammoniumchlorid (AKZO) | |
| ²³ ethoxyliertes Methylglucosid-dioleat (CTFA-Bezeichnung: PEG-120 Methyl Glucose Dioleate) (AMERCHOL) | |

| 5) Shampoo | |
|---|---|
| Texapon^{R}K 14 S²⁸ | 50,0 |
| Dehyton^{R}K | 10,0 |
| Akypo^{R}RLM 100 NV²⁹ | 4,5 |
| Cutina^{R}AGS³⁰ | 2,0 |
| D-Panthenol | 0,5 |
| Glucose | 1,0 |
| Salicylsäure | 0,4 |
| Natriumchlorid | 0,5 |
| Polymer P1, gemäß DE 39 29 973 | 0,6 |
| Luviquat^{R}HM 552 | 1,0 |
| Wasser | ad 100 |

| | |
|---|---|
| ²⁸ Natriumlaurylmyristylethersulfat (ca. 28 % Aktivsubstanz; CTFA-Bezeichnung: Sodium Myreth Sulfate) (HENKEL) | |
| ²⁹ C12-14-Fettalkohol+10 Ethylenoxid-essigsäure-Natriumsalz (22 % Aktivsubstanz; CTFA-Bezeichnung: Sodium Laureth-11 Carboxylate) (CHEM-Y) | |
| ³⁰ Ethylenglykolstearat (ca. 5-15% Monoester, 85-95% Diester; CTFA-Be zeichnung: Glycol Distearate) (HENKEL) | |

| 6) Shampoo | |
|---|---|
| Texapon^{R}K 14 S | 25,0 |
| Texapon^{R}SB 3³¹ | 7,5 |
| Eucarol^{R}TA³² | 12,0 |
| Akypo^{R}RLM 100 NV | 9,0 |
| Dehyton^{R}AB 30³³ | 8,3 |
| Elfacos^{R}GT 282S³⁴ | 0,5 |
| Natriumchlorid | 0,5 |
| Polymer P1, gemäß DE 39 29 973 | 0,6 |
| Luviquat^{R}FC 550 | 4,0 |
| Wasser | ad 100 |

| | |
|---|---|
| ³¹ Sulfobernsteinsäurehalbester auf Basis eines Alkylpolyglycolethers, Di-Na-Salz (ca. 40 % Aktivsubstanz; CTFA-Bezeichnung: Disodium Laurethsulfosuccinat) (HENKEL) | |
| ³² Laurylalkohol+7 Ethylenoxid-tartrat-Natriumsalz (ca. 25 % Aktivsubstanz; CTFA-Bezeichnung: Sodium Laureth-7 Tartrate) (AUSICHEM) | |
| ³³ Fettamin-Derivat mit Betainstruktur (ca. 30 % Aktivsubstanz; CTFA-Bezeichnung: Coco-Betaine) (HENKEL) | |
| ³⁴ Talgalkohol+60 Ethylenoxid-myristylether (CTFA-Bezeichnung: Talloweth-60 Myristyl Glycol) (AKZO) | |

| 7) Kurpackung (abspülbar) | |
|---|---|
| Stenol^{R}1618 | 3,0 |
| Eumulgin^{R}B 1 | 0,5 |
| Eumulgin^{R}B 2 | 0,5 |
| Cutina^{R}CP¹⁰ | 1,0 |
| Eutanol^{R}G | 1,5 |
| Carbopol 980³⁵ | 0,004 |
| Dow Corning^{R}929-Emulsion ¹³ | 2,9 |
| Polymer P1, gemäß DE 39 29 973 | 0,3 |
| Luviquat^{R}FC 550 | 4,0 |
| Triethanloamin | 0,09 |
| Wasser | ad 100 |

| | |
|---|---|
| ¹⁰ Ester aus gesättigten, langkettigen Fettalkoholen und Fettsäuren, vornehmlich Palmitinsäurecetylester (CTFA-Bezeichnung: Cetyl Palmi tate) (HENKEL) | |
| ¹³ amino-funktionelles Polydimethylsiloxan (35 % Aktivsubstanz:) (DOW CORNING) | |
| ³⁵ Polyacrylsäure (GOODRICH) | |

| 8) Färbecreme | |
|---|---|
| C12-18-Fettalkohol | 1,2 |
| Lanette^{R}O | 4,0 |
| Eumulgin^{R}B 2 | 0,8 |
| Cutina^{R}KD 16⁴¹ | 2,0 |
| Natriumsulfit | 0,5 |
| L(+)-Ascorbinsäure | 0,5 |
| Ammoniumsulfat | 0,5 |
| 1,2-Propylenglykol | 1,2 |
| Polymer JR^{R}400⁴² | 0,3 |
| p-Aminophenol | 0,35 |
| p-Toluylendiamin | 0,85 |
| 2-Methylresorcin | 0,14 |
| 6-Methyl-m-aminophenol | 0,42 |
| Polymer P1, gemäß DE 39 29 973 | 0,7 |
| Luviquat^{R}FC 550 | 1,0 |
| Ammoniak | 1,5 |
| Wasser | ad 100 |

| | |
|---|---|
| ⁴¹ Fettsäuremono/diglycerid-Emulgator-Gemisch (CTFA-Bezeichnung: Tallow Glycerides (and) Glyceryl Stearate (and) Potassium Stearate) (HENKEL) | |
| ⁴² quaternierte Hydroxyethylcellulose (Union Carbide) | |

| 9) Entwickleremulsion für Färbecreme 11) | |
|---|---|
| Texapon^{R}N 25 | 2,1 |
| Wasserstoffperoxid (50%ig) | 12,0 |
| Turpinal^{R}SL⁴³ | 1,7 |
| Latekoll^{R}D⁴⁴ | 12,0 |
| Polymer P1, gemäß DE 39 29 973 | 0,1 |
| Luviquat^{R}FC 550 | 0,9 |
| Wasser | ad 100 |

| | |
|---|---|
| ⁴³ 1-Hydroxyethan-1,1-diphosphonsäure (60 % Aktivsubstanz; CTFA-Bezeichnung: Etidronic Acid) (HENKEL) | |
| ⁴⁴ Acrylester-Methacrylsäure-Copolymer (25 % Aktivsubstanz) (BASF) | |

Die Färbecreme hatte einen pH-Wert von 10,0. Sie bewirkte eine intensive rote Tönung des Haares.

| 10) Cremedauerwelle Wellcreme: | |
|---|---|
| Plantaren^{R}-800⁴⁷ | 5,0 |
| Thioglykolsäure | 8,0 |
| Turpinal^{R}SL | 0,5 |
| Ammoniak (25%ig) | 7,3 |
| Ammoniumcarbonat | 3,0 |
| Cetyl/Stearyl-Alkohol | 5,0 |
| Guerbet-Alkohol | 4,0 |
| Luviquat^{R}HM 552 | 1,5 |
| Polymer P1, gemäß DE 39 29 973 | 0,1 |
| Parfümöl | q.s. |
| Wasser | ad 100 |

| | |
|---|---|
| ⁴⁷ C8-C10-Alkylglucosid mit Oligomerisationsgrad 1,6 (ca. 60% Aktivsubstanz) (HENKEL) | |

| Fixierlösung: | |
|---|---|
| Plantaren^{R}-800 | 5,0 |
| gehärtetes Rizinusöl | 2,0 |
| Kaliumbromat | 3,5 |
| Nitrilotriessigsäure | 0,3 |
| Zitronensäure | 0,2 |
| Polymer P1, gemäß DE 39 29 973 | 0,2 |
| Luviquat^{R}FC 550 | 1,0 |
| Parfümöl | q.s. |
| Wasser | ad 100 |

## Patentansprüche

1. Wäßrige Zubereitung zur Behandlung von Haaren, enthaltend neben üblichen kosmetischen Bestandteilen eine Kombination von kationischen und amphoteren oder zwitterionischen Polymeren, dadurch gekennzeichnet, daß die kationischen Polymeren Imidazoliniumgruppen enthalten sowie mindestens ein nichtionisches Monomer, wobei das Verhältnis zwischen kationischen und nichtionischen Monomeren im Bereich 1,5:1 bis 1:1,5 liegt, und sich die zwitterionischen Polymeren im wesentlichen zusammensetzen aus
α) Monomeren mit quartären Ammoniumgruppen der allgemeinen Formel (I),
R¹-CH=CR²-CO-Z-(CₙH₂ₙ)-N⁽⁺⁾R³R⁴R⁵ A⁽⁻⁾ (I)
in der R¹ und R² unabhängig voneinander stehen für Wasserstoff oder eine Methylgruppe und R³, R⁴ und R⁵ unabhängig voneinander für Alkylgruppen mit 1 bis 4 Kohlenstoff-Atomen, Z eine NH-Gruppe oder ein Sauerstoffatom, n eine ganze Zahl von 2 bis 5 und A⁽⁻⁾ das Anion einer organischen oder anorganischen Säure ist
und
β) monomeren Carbonsäuren der allgemeinen Formel (II),
R⁶-CH=CR⁷-COOH (II)
in denen R⁶ und R⁷ unabhängig voneinander Wasserstoff oder Methylgruppen sind, oder Alkali-, Erdalkali-, Aluminium- oder Ammoniumsalzen dieser Säuren.

2. Zubereitung nach Anspruch 1, dadurch gekennzeichnet, daß das nichtionische Monomere Vinylpyrrolidon ist.

3. Zubereitung nach Anspruch 1 oder 2, dadurch gekennzeichnet, daß das Monomere des Typs (α) Acrylamidopropyl-trimethyl-ammoniumchlorid und das Monomere des Typs (β) Acrylsäure oder ein Alkalisalz, insbesondere das Natriumsalz, der Acrylsäure ist.

4. Zubereitung nach einem der Ansprüche 1 bis 3, dadurch gekennzeichnet, daß im zwitterionischen Polymer die Zahl der Monomeren vom Typ (α) größer als die Zahl der Monomeren vom Typ (β) ist.

5. Zubereitung nach einem der Ansprüche 1 bis 4, dadurch gekennzeichnet, daß sie
- 0,01 - 10, insbesondere 0,01 bis 5 Gew.-%, zwitterionischen Polymeren und
- 0,01 - 10, insbesondere 0,01 bis 5 Gew.-%, an kationischen Polymeren, jeweils bezogen auf die gesamte Zubereitung, enthält.

6. Zubereitung nach einem der Ansprüche 1 bis 5, dadurch gekennzeichnet, daß sie zusätzlich ein Tensid enthält.

7. Zubereitung nach Anspruch 6, dadurch gekennzeichnet, daß das Tensid ein kationisches oder nichtionisches Tensid ist.

8. Zubereitung nach einem der Ansprüche 1 bis 7, dadurch gekennzeichnet, daß die Zubereitung einen pH-Wert zwischen 2,5 und 7, insbesondere zwischen 3,0 und 6,0 aufweist.

9. Verfahren zur Behandlung von Haaren, dadurch gekennzeichnet, daß eine Zubereitung nach einem der Ansprüche 1 bis 8 auf das Haar aufgebracht wird.

10. Verfahren nach Anspruch 9, dadurch gekennzeichnet, daß das Haar anschließend mit Wasser oder einem im wesentlichen Wasser enthaltenden Mittel gespült wird.

## Claims

1. A water-based hair treatment preparation containing a combination of cationic and amphoteric or zwitterionic polymers in addition to typical cosmetic constituents, characterized in that the cationic polymers contain imidazolinium groups and at least one nonionic monomer, the ratio between cationic and nonionic monomers being in the range from 1.5:1 to 1:1.5, and the zwitterionic polymers consist essentially of
(α) monomers containing quaternary ammonium groups corresponding to general formula (I):
R¹-CH=CR²-CO-Z-(CₙH₂ₙ)-N⁽⁺⁾R³R⁴R⁵ A⁽⁻⁾ (I)
in which R¹ and R² independently of one another represent hydrogen or a methyl group and R³, R⁴ and R⁵ independently of one another represent C₁₋₄ alkyl groups, Z is an NH group or an oxygen atom, n is an integer of 2 to 5 and A⁽⁻⁾ is the anion of an organic or inorganic acid
and
(β) monomeric carboxylic acids corresponding to general formula (II):
R⁶-CH=CR⁷-COOH (II)
in which R⁶ and R⁷ independently of one another are hydrogen or methyl groups, or alkali metal, alkaline earth metal, aluminium or ammonium salts of these acids.

2. A preparation as claimed in claim 1, characterized in that the nonionic monomer is vinyl pyrrolidone.

3. A preparation as claimed in claim 1 or 2, characterized in that the monomer (α) is acrylamidopropyl trimethyl ammonium chloride while the monomer (β) is acrylic acid or an alkali metal salt, more particularly the sodium salt, of acrylic acid.

4. A preparation as claimed in any of claims 1 to 3, characterized in that the number of monomers (α) in the zwitterionic polymer is greater than the number of monomers (β).

5. A preparation as claimed in any of claims 1 to 4, characterized in that it contains
- 0.01 to 10% by weight and more particularly 0.01 to 5% by weight of zwitterionic polymers and
- 0.01 to 10% by weight and more particularly 0.01 to 5% by weight of cationic polymers, based on the preparation as a whole.

6. A preparation as claimed in any of claims 1 to 5, characterized in that it also contains a surfactant.

7. A preparation as claimed in claim 6, characterized in that the surfactant is a cationic or nonionic surfactant.

8. A preparation as claimed in any of claims 1 to 7, characterized in that the preparation has a pH value of 2.5 to 7 and, more particularly, in the range from 3.0 to 6.0.

9. A process for treating hair, characterized in that a preparation as claimed in any of claims 1 to 8 is applied to the hair.

10. A process as claimed in claim 9, characterized in that the hair is subsequently rinsed with water or with a preparation largely containing water.

## Revendications

1. Préparation aqueuse pour le traitement capillaire, renfermant à côté des constituants cosmétiques usuels, une combinaison de polymères cationiques et amphotères ou zwitterioniques,
caractérisée en ce que
les polymères cationiques renferment des groupes imidazolinium ainsi qu'au moins un monomère non ionique, dans laquelle le rapport entre monomères cationiques et non ioniques se situe dans la zone de 1,5:1 à 1:1,5 et en ce que les polymères zwitterioniques se composent essentiellement de :
α) des monomères avec des groupes ammonium quaternaires de formule générale (I),
R¹-CH=CR²-CO-Z-(CₙH₂ₙ)-N⁽⁺⁾R³R⁴R⁵ A⁽⁻⁾ (I)
dans laquelle R¹ et R², indépendamment l'un de l'autre, représentent de l'hydrogène, ou un groupe méthyle et R³, R⁴ et R⁵ indépendamment l'un de l'autre, représentent des groupes alkyle ayant de 1 à 4 atomes de carbone, Z représente un groupe -NH ou un atome d'oxygène, n est un nombre entier allant de 2 à 5 et, A⁽⁻⁾ est l'anion d'un acide organique ou minéral et,
β) des acides carboxyliques monomères de formule générale (II)
R⁶-CH=CR⁷-COOH (II)
dans laquelle R⁶ et R⁷, indépendamment l'un de l'autre, sont de l'hydrogène ou un groupe méthyle, ou les sels de métal alcalin, alcalino-terreux, d'aluminium ou d'ammonium de ces acides.

2. Préparation selon la revendication 1,
caractérisée en ce que
le monomère non ionique est la vinylpyrrolidone.

3. Préparation selon la revendication 1 ou la revendication 2,
caractérisée en ce que
le monomère de type (α) est le chlorure d'acrylamidopropyltriméthylammonium et le monomère de type (β) est l'acide acrylique ou un sel de métal alcalin, en particulier le sel de sodium, de l'acide acrylique.

4. Préparation selon l'une des revendications 1 à 3,
caractérisée en ce que
dans le polymère zwitterionique, le nombre de monomères du type (α) est plus grand que le nombre de monomères de type (β).

5. Préparation selon une des revendications 1 à 4,
caractérisée en ce qu'
elle contient de 0,01 à 10 en particulier de 0,01 à 5 % en poids de polymères zwitterioniques et de 0,01 à 10 en particulier de 0,01 à 5 % en poids de polymères cationiques, à chaque fois rapporté à la préparation totale.

6. Préparation selon l'une des revendications 1 à 5,
caractérisée en ce qu'
elle contient en supplément un agent tensioactif.

7. Préparation selon la revendication 6,
caractérisée en ce que
l'agent tensioactif est un agent tensioactif cationique ou non-ionique.

8. Préparation selon l'une des revendications 1 à 7,
caractérisée en ce que
la préparation possède une valeur de pH comprise entre 2,5 et 7, en particulier entre 3,0 et 6,0.

9. Procédé de traitement capillaire,
caractérisé en ce qu'
une préparation selon l'une des revendications 1 à 8 est appliquée sur le cheveu.

10. Procédé selon la revendication 9,
caractérisé en ce que
le cheveu ensuite est rincé avec de l'eau ou avec un moyen contenant essentiellement de l'eau.
